# EUROPÄISCHE PATENTSCHRIFT

(11) **EP 3 363 399 B1**
(45) Veröffentlichungstag und Bekanntmachung des Hinweises auf die Patenterteilung: **25.03.2020**
(21) Anmeldenummer: 18156281.0
(22) Anmeldetag: 12.02.2018
(51) Int. Cl.: A61B 34/00, A61B 90/00, A61B 34/30

(54) **BEDIENGERÄT FÜR EIN ROBOTERGESTÜTZTES CHIRURGIESYSTEM**
CONTROLLER FOR A ROBOT-ASSISTED SURGICAL SYSTEM
UNITÉ DE COMMANDE POUR UN SYSTÈME CHIRURGICAL ASSISTÉ PAR ROBOT

(30) Priorität: 16.02.2017 DE 102017103199
(43) Veröffentlichungstag der Anmeldung: 22.08.2018
(73) Patentinhaber: avateramedical GmbH, 07745 Jena (DE)
(72) Erfinder: Ziegenspeck, Nils, 70176 Stuttgart (DE); Trommer, Christian, 99310 Wipfratal/Schmerfeld (DE); Gude, Geralf, 17258 Feldberger Seenlandschaft (DE); Schrumpf, Thomas, 99096 Erfurt (DE); Karguth, Andreas, 99869 Tüttleben (DE)
(74) Vertreter: Schaumburg und Partner Patentanwälte mbB

(56) Entgegenhaltungen:
- EP-A1- 2 740 434
- US-A1- 2009 171 374
- US-A1- 2010 228 265
- US-B1- 6 424 885
- US-B1- 6 799 065

## Beschreibung

Die Erfindung betrifft ein Bediengerät für ein robotergestütztes Chirurgiesystem, das ein manuell betätigbares Bedienelement umfasst, das zwei Laschen zur Aufnahme jeweils eines Fingers einer Bedienperson, insbesondere zur Aufnahme des Daumens und des Mittelfingers der Bedienperson, umfasst. Ferner hat das Bediengerät eine Schnittstelle zur Befestigung an einer Halteeinheit, wobei das Bediengerät, wenn es über diese Schnittstelle an der Halteeinheit befestigt ist, relativ zur Schnittstelle um eine erste, eine zweite und eine dritte Drehachse drehbar ist, wobei diese drei Drehachsen orthogonal zueinander gerichtet sind.

In der minimalinvasiven Chirurgie werden zunehmend sogenannte Telemanipulatorsysteme, die auch als Roboterassistenzsysteme oder allgemein als Vorrichtung zur robotergestützten Chirurgie bezeichnet werden, eingesetzt. Mit Hilfe einer Vorrichtung zur robotergestützten Chirurgie werden chirurgische Instrumente aufgrund von Bedieneingaben in ihrer Lage und Orientierung gesteuert. Die chirurgischen Instrumente werden ferner mechanisch, elektrisch und/oder optisch an das Telemanipulatorsystem angekoppelt, um eine aktive Positionierung und Ausrichtung des chirurgischen Instruments sowie einer gewünschten Betätigung eines chirurgischen Instruments realisieren zu können. Hierzu haben die chirurgischen Instrumente, die neben Instrumenten mit Endeffektoren auch Endoskope und zu bedienende medizinische Geräte umfassen, eine Koppelschnittstelle, die als Koppeleinheit ausgebildet sein kann und auch als Sterileinheit bezeichnet wird. Die Vorrichtung zur robotergestützten Chirurgie hat ferner mindestens einen Manipulatorarm, an dessen proximalen Ende die Koppeleinheit vorgesehen ist, mit der die Sterileinheit verbindbar ist, um die mechanische, elektrische und/oder optische Kopplung zwischen dem Manipulatorarm und dem chirurgischen Instrument zu ermöglichen.

Zur Steuerung des Telemanipulatorsystems werden Bediengeräte verwendet, die an einer zentralen Steuereinheit angeordnet sind, und die dem Chirurgen eine möglichst genaue Steuerung der an dem Roboterarm befestigten chirurgischen Instrumente ermöglichen soll.

US 6424885 B1 offenbart ein solches Bediengerät zur Steuerung eines Telemanipulatorsystems.

Problematisch bei bekannten Bediengeräten ist insbesondere, dass diese häufig nicht eine intuitive Bewegung der chirurgischen Instrumente ermöglichen, insbesondere die Bewegung nicht derart erfolgt, als ob der Chirurg das chirurgische Instrument tatsächlich direkt in seiner Hand halten würde. Ursache hierfür ist in der Regel, dass die einzelnen Bewegungs- und Drehachsen der Bediengeräte von denen der menschlichen Hand deutlich abweichen, so dass eine abweichende Bewegung von der Hand erfolgt. Dies hat zur Konsequenz, dass sich der Chirurg jeweils an die Eingabegeräte gewöhnen muss, und ohne ausreichendes Training die notwendige präzise Steuerung der chirurgischen Instrumente nicht gewährleistet werden kann.

Es ist Aufgabe der Erfindung, ein Bediengerät für ein robotergestütztes Chirurgiesystem anzugeben, mit dessen Hilfe das robotergestützte Chirurgiesystem möglichst genau gesteuert werden kann.

Diese Aufgabe wird durch ein Bediengerät mit den Merkmalen den Anspruchs 1 gelöst. Vorteilhafte Weiterbildungen sind in den abhängigen Ansprüchen angegeben. Erfindungsgemäß ist das Bediengerät derart ausgebildet, dass sich die drei Drehachsen, um die das Bedienelement drehbar ist, in einem gemeinsamen Schnittpunkt schneiden.

Dieser Schnittpunkt liegt hierbei insbesondere innerhalb des Bedienelements, wobei der Schnittpunkt insbesondere zwischen den beiden Laschen, durch die die Finger hindurchgeführt sind, angeordnet ist. Insbesondere ist der Schnittpunkt mittig zwischen den beiden Laschen und vorzugsweise derart angeordnet, dass er mit dem Mittelpunkt des Bedienelements zusammenfällt.

Es hat sich gezeigt, dass durch die Anordnung der Drehachsen derart, dass sie sich in einem gemeinsamen Punkt schneiden, und insbesondere dass dieser zwischen den Laschen und somit zwischen den Fingern des Chirurgen angeordnet ist, sich eine besonders intuitive, einfache Steuerung ergibt, da der so folgende Griff und die über das Bediengerät erfassten Bewegungen intuitiv denen entsprechen, als ob der Chirurg mit seinen beiden Fingern, die durch Laschen geführt sind, das chirurgische Instrument direkt fassen würde.

Unter Laschen wird insbesondere jede Art von Schlaufe oder anderen Elementen verstanden, durch die die Finger hindurchgeführt werden können. Insbesondere ist das Bedienelement derart ausgebildet, dass durch die eine Lasche der Daumen des Chirurgen und durch die andere Lasche der Mittelfinger geführt ist werden kann. Insbesondere sind die Laschen aus einem flexiblen Element, beispielsweise einem Band ausgebildet.

Darüber hinaus hat die Anordnung der drei Drehachsen derart, dass sie sich in einem Punkt schneiden und dieser innerhalb des Bedienelements liegt den Vorteil, dass hierdurch das auszugleichende Massenträgheitsmoment und die parasitären Kräfte minimiert werden, wodurch eine noch einfachere intuitive Steuerung erreicht wird.

Das Bediengerät umfasst insbesondere ein Basiselement, das die Schnittstelle umfasst, so dass das Bediengerät über dieses Basiselement an der Halteeinheit befestigbar ist, wobei dieses Basiselement dann insbesondere drehfest an der Halteeinheit angeordnet ist. Bei der Halteeinheit kann es sich insbesondere um einen Arm eines Stativs handeln, wobei dieser Arm wiederum insbesondere in bis zu drei Richtungen translatorisch bewegbar und zusätzlich um ein Gelenk in bis zu drei Richtungen drehbar sein kann. Hierdurch wird erreicht, dass eine Ausrichtung in möglichst jeder beliebiger Position innerhalb des Raums möglich ist.

An dem Basiselement ist insbesondere eine Schwenkeinheit drehbar gelagert, wobei die Lagerung derart erfolgt, dass die Schwenkeinheit relativ zum Basiselement um die erste Drehachse drehbar gelagert ist. An der Schwenkeinheit wiederum ist relativ zur Schwenkeinheit um die zweite Achse drehbar ein Gehäuse befestigt, wobei an diesem Gehäuse dann wiederum das Bedienelement um die dritte Drehachse drehbar befestigt ist. Über diese Kette von drehbaren Befestigungen wird erreicht, dass das Bedienelement, welches der Chirurg zur Bedienung in seiner Hand hält, um alle drei Drehachsen relativ zur Schnittstelle drehbar ist.

Die Bewegung, die der Chirurg am Bedienelement ausführt wird insbesondere mithilfe von Sensoren ermittelt, wobei das robotergestützte Chirurgiesystem anschließend derart angesteuert wird, dass die von ihm gehaltenen chirurgischen Instrumente die zuvor ermittelte Bewegung ausführen.

Bei der Erfindung ist das Bedienelement scheibenförmig ausgebildet, wobei unter scheibenförmig insbesondere ein in etwa zylinderförmiges Element verstanden wird, dessen Durchmesser größer als seine Dicke ist.

Die beiden Laschen sind insbesondere an den beiden Seiten der Scheibe einander gegenüberliegend angeordnet.

Ein solches scheibenförmiges Element kann auf besonders einfache Weise und ergonomisch günstig innerhalb der Hand des Chirurgen aufgenommen werden, wobei insbesondere die Handinnenfläche auf der Umfangsfläche des Bedienelements aufliegt.

Das Gehäuse weist insbesondere zwei Ringe auf, zwischen denen das scheibenförmige Bedienelement angeordnet ist, wobei das Bedienelement über Lager, insbesondere Dünnringlager, an dem Gehäuse befestigt ist, so dass das Bedienelement relativ zu dem Gehäuse um die dritte Achse gedreht werden kann. Insbesondere erfolgt die Lagerung derart, dass eine Drehung um 360 Grad möglich ist.

Das Gehäuse wiederum ist über weiteres Lager, insbesondere ein Kugellager, vorzugsweise zwei Dünnringlager, an der Schwenkeinheit drehbar befestigt. Somit wird auch hierdurch eine Drehung um 360 Grad relativ zur Schwenkeinheit erreicht, was im Zusammenhang mit der Lagerung des Bedienelements ermöglicht, dass das Bedienelement relativ zur Schwenkeinheit und somit auch zur Schnittstelle sowohl um die zweite als auch um die dritte Drehachse um 360 Grad drehbar ist.

Die Schwenkeinheit wiederum ist über mindestens ein Kugellager, vorzugsweise zwei Dünnringlager drehbar an dem Basiselement befestigt.

Ferner ist es vorteilhaft, wenn das Bediengerät einen ersten Aktor zum Drehen der Schwenkeinheit um die erste Achse, einen zweiten Aktor zum Drehen des Gehäuses um die zweite Achse und/oder einen dritten Aktor zum Drehen des Bedienelements um die dritte Drehachse umfasst. Durch diese Aktoren kann zum einen das Ausrichten des Bedienelements in einer voreingestellten Nullposition erfolgen, wenn das Bedienelement zuvor aus dieser Nullposition herausbewegt worden ist. Zum anderen kann über diese Aktoren eine Kraftrückkopplung auf das Bedienelement, ein sog. Force Feedback, erfolgen. Hierzu werden insbesondere über Sensoren die auf die chirurgischen Instrumente während der Operation einwirkenden Kräfte und/oder Drehmomente bestimmt und über die Aktoren auf das Bedienelement zurückgekoppelt, so dass der Chirurg Widerstände und Kräfte der chirurgischen Instrumente erfahren kann und somit das Gefühl erhält, als ob er direkt die chirurgischen Instrumente halten würde.

Bei den Aktoren handelt es sich insbesondere um Motoren, vorzugsweise Elektromotoren. Hierdurch werden einfacher Aufbau und eine präzise Ansteuerung ermöglicht.

Der erste Aktor ist insbesondere am Basiselement angeordnet und vorzugsweise über ein Zahnriemengetriebe mit der Schwenkeinheit gekoppelt, so dass das Basiselement um die erste Drehachse gedreht werden kann.

Der zweite Aktor ist insbesondere an der Schwenkeinheit angeordnet und vorzugsweise über ein weiteres Zahnradgetriebe mit dem Gehäuse gekoppelt, so dass das Gehäuse über den zweiten Aktor um die zweite Drehachse relativ zur Schwenkeinheit gedreht werden kann.

Der dritte Aktor dagegen ist insbesondere nicht am Gehäuse, sondern am Bedienelement angeordnet und wird zusammen mit diesem Bedienelement jeweils mitgedreht. Auf diese Weise wird erreicht, dass das Bedienelement relativ zum Gehäuse um 360° beliebig gedreht werden kann.

Der dritte Aktor ist insbesondere über ein Ritzel und ein Kronenrad mit dem Gehäuse verbunden, so dass eine Drehbarkeit relativ zu dem Gehäuse um die dritte Drehachse gegeben ist.

Ferner weist das Bediengerät insbesondere einen ersten Sensor zur Ermittlung der Drehstellung um die erste Drehachse, einen zweiten Sensor zur Ermittlung der Drehstellung um die zweiten Achse und/oder einen dritten Sensor zur Ermittlung der Drehstellung um die dritte Achse auf, wobei die hierdurch gewonnenen Informationen insbesondere zur Ansteuerung des Robotersystems genutzt werden. Insbesondere können die Sensoren jeweils auch in die jeweiligen Aktoren integriert sein bzw. die in den Aktoren ohnehin vorgesehenen Sensoren hierfür verwendet werden.

Ferner ist es alternativ möglich, dass ein Sensor zur Bestimmung der Drehstellung um mehrere Achsen verwendet wird.

Das Bediengerät ist insbesondere derart ausgebildet, dass das Bedienelement sowohl um die zweite als auch um die dritte Drehachse um 360° drehbar ist. Hierdurch wird erreicht, dass ein möglichst großer Verstellbereich gewährleistet ist.

Bei einer bevorzugten Ausführungsform der Erfindung weist das Bedienelement einen Taster auf, über den die Bedienperson weitere Funktionen des robotergestützten Chirurgiesystems steuern kann. Dieses Bedienelement kann insbesondere als ein Schiebetaster ausgebildet sein und ist insbesondere auf der Umfangfläche des scheibenförmigen Bedienelements angeordnet.

Insbesondere ist der Schiebetaster mithilfe eines elastischen Elements, beispielsweise einer Druckfeder, in einer vorbestimmten Stellung vorgespannt und kann von der Bedienperson entgegen der Federkraft zur Betätigung eines Mikroschalters bewegt werden. Durch die Rückstellkraft wird der Taster nach dem Loslassen wieder an seine Ausgangsposition bewegt.

Alternativ kann anstelle eines Tasters beispielsweise auch ein Schalter oder ein Knopf zur Steuerung vorgesehen werden.

Der Taster, der Schalter oder der Knopf können vorzugsweise zusammen mit den an beiden Seiten des Grundkörpers des Bedienelements angeordneten Laschen drehbar sein, sodass der Chirurg ihn bequem jederzeit unabhängig von der Stellung der Laschen erreichen und bedienen kann.

Bei einer besonders bevorzugten Ausführungsform sind die beiden Laschen derart an dem scheibenförmigen Grundelement des Bedienelements befestigt, dass durch sie eine Art Pinzettengriff gebildet wird, bei dem der Chirurg die Möglichkeit hat, seinen beiden durch die Laschen geführten Finger zu spreizen und zusammenzuführen, und diese Bewegung durch entsprechende Sensoren erfasst wird und bei der Ansteuerung des chirurgischen Instruments des robotergestützten Chirurgiesystems berücksichtigt wird.

Hierzu sind die beiden Laschen insbesondere um einen vorbestimmten Winkel um vorbestimmte Drehpunkte voneinander wegschwenkbar, wobei die beiden Laschen insbesondere derart gekoppelt sind, dass sie relativ zu einer Nullposition immer beide um den gleichen Winkel geöffnet sind. Diese Nullposition ist insbesondere diejenige Position, bei der die beiden Flächen des scheibenförmigen Bedienelements, an denen die Laschen befestigt sind, zueinander parallel ausgerichtet sind.

Weitere Merkmale und Vorteile der Erfindung ergeben sich aus der folgenden Beschreibung, die die Erfindung anhand von Ausführungsbeispielen im Zusammenhang mit den beigefügten Figuren näher erläutert.

Es zeigen:
- Figur 1: eine schematische Seitenansicht eines Systems zur robotergestützten Chirurgie mit einem Manipulator, der vier Manipulatorarme hat, mit denen jeweils eine Instrumenteneinheit verbindbar ist,
- Figur 2: eine schematische Darstellung eines Bediengeräts zur Steuerung des Manipulators nach Figur 2 sowie eine Halteeinheit, an der das Bediengerät befestigt ist,
- Figur 3: eine schematische, perspektivische Darstellung des Bediengeräts nach Figur 2,
- Figur 4: eine Draufsicht auf das Bediengerät nach den Figuren 2 und 3 bei geöffnetem Pinzettengriff,
- Figur 5: eine Schnittdarstellung durch das Bediengerät nach den Figuren 2 bis 4,
- Figur 6: einen Ausschnitt einer Schnittdarstellung eines Bedienelements des Bediengeräts nach den Figuren 2 bis 5, und
- Figur 7 u. 8: jeweils eine schematische Darstellung eines Ausschnitts des Bedienelements.

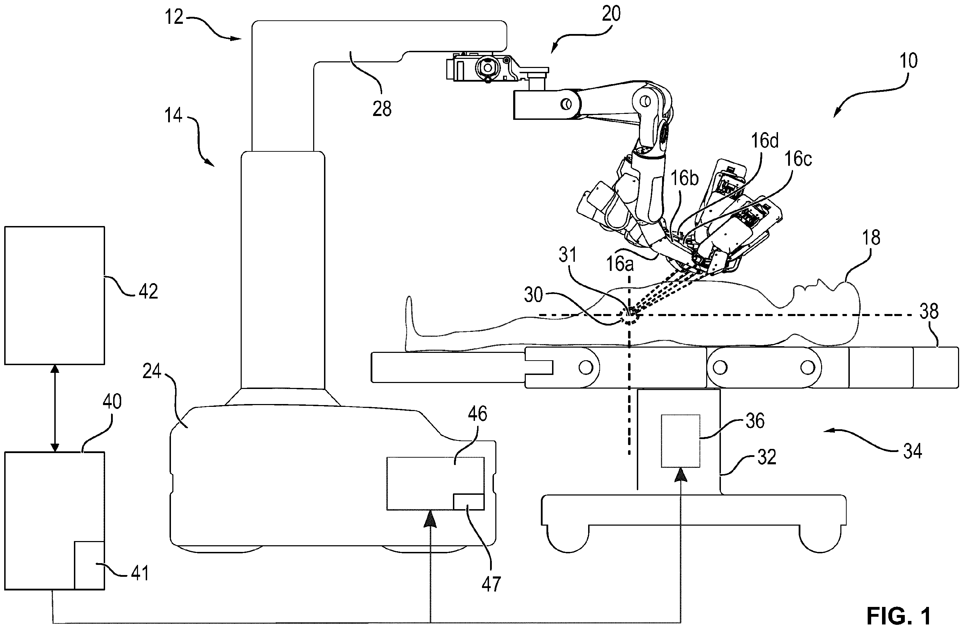

Figur 1 zeigt eine schematische Seitenansicht eines Systems 10 zur robotergestützten Chirurgie mit einem Manipulator 12, der ein Stativ 14 und vier Manipulatorarme 16a bis 16d hat. Der Manipulator 12 wird allgemein auch als Vorrichtung zur robotergestützten Chirurgie bezeichnet. Das System 10 dient zur Durchführung eines chirurgischen Eingriffs an einem auf einem Operationstisch 34 positionierten Patienten 18. Ausgehend von der Anatomie des Patienten 18 und der durchzuführenden Operation sind die Koordinaten eines Zieloperationsgebiets 30 ermittelt und voreingestellt gespeichert worden. Das Stativ 14 hat einen L-förmigen Stativarm 28, an dessen vom Stativfuß 24 entfernten Ende die Manipulatorarme 16a bis 16d über einen Stativkopf 20 verbunden sind.

Der Operationstisch 34 hat eine Operationstischsäule 32, in der eine Steuereinheit 36 des Operationstischs 34 angeordnet ist und auf der eine mehrere Segmente umfassende Patientenlagerfläche 38 angeordnet ist. Die Steuereinheit 36 dient zur Steuerung der Bewegung von Elementen des Operationstischs 34, insbesondere zur Längenverstellung der Operationstischsäule 32 und somit zum Verstellen der Höhe der Patientenlagerfläche 38 und zum Verstellen einzelner Segmente sowie der Neigung und der Kantung der Patientenlagerfläche 38. Vorzugsweise ist jedoch die Verstellung der Segmente des Operationstischs 34 während eines operativen Eingriffs mit Hilfe des Manipulators 12 gesperrt. Das System 10 umfasst ferner eine Steuereinheit 46 des Manipulators 12 sowie eine zentrale Steuereinheit 40, die über Datenleitungen mit der Steuereinheit 46 des Manipulators 12, der Steuereinheit 36 des Operationstischs 34 sowie einer Bedienkonsole 42 mit einer Anzeigeeinheit 44 verbunden ist. Die Steuereinheit 40 hat eine Ausgabeeinheit 41 und die Steuereinheit 46 hat eine Ausgabeeinheit 47, durch die jeweils optische und/oder akustische Signale ausgegeben werden können.

Die Oberfläche der Patientenlagerfläche 38 bildet eine Frontalebene, auf der der Patient 18 dorsal liegend positioniert ist.

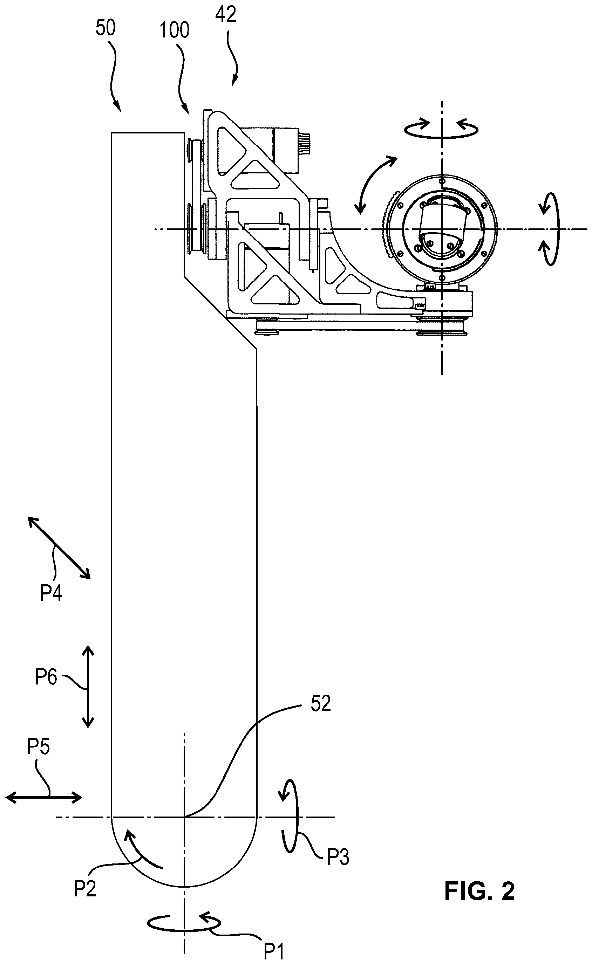

In Figur 2 ist eine schematische Darstellung des Bediengerät 42 sowie eines Haltearms 50, an dem das Bediengerät 42 über eine Schnittstelle 100 des Bediengeräts befestigt ist, dargestellt.

Der Haltearm 50 ist um einen Drehpunkt 52, wie durch die Pfeile P1 bis P3 angedeutet, um drei Achsen, die orthogonal zueinander sind, drehbar. Darüber hinaus kann der Haltearm 50 translatorisch in drei orthogonal zueinander gerichtete Richtungen, wie durch die Pfeile P4 bis P6 angedeutet, bewegt werden. Somit kann das Bediengerät 42 in einer ergonomisch günstigen Position für die Bedienperson ausgerichtet werden.

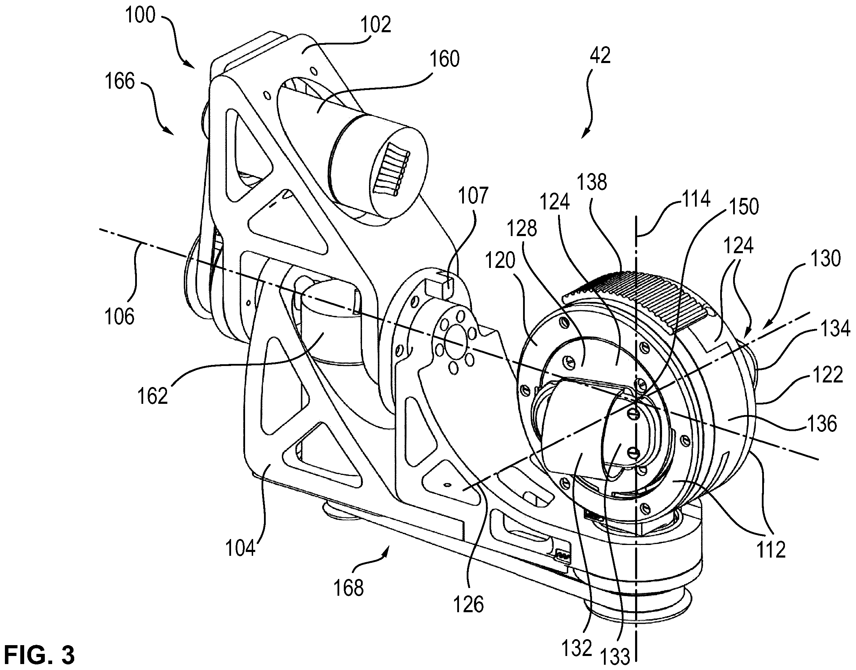

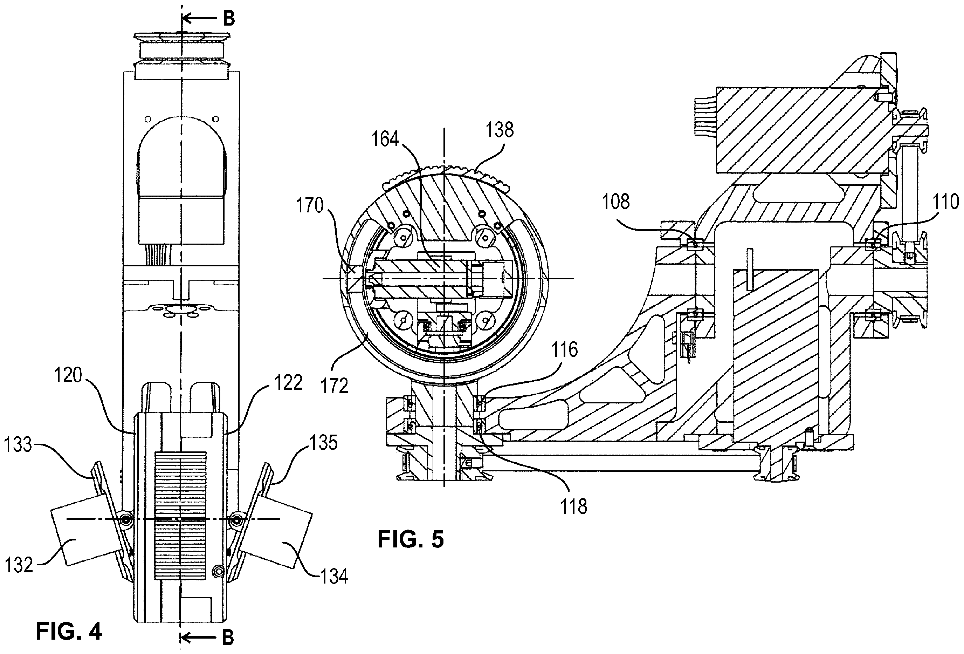

In Figur 3 ist eine schematische, perspektivische Darstellung des Bediengeräts 42 gezeigt. Figur 4 zeigt eine Draufsicht auf das Bediengerät und Figur 5 eine Schnittdarstellung durch das Bediengerät 42 gemäß des Schnitts B-B nach Figur 4.

Das Bediengerät 42 umfasst ein Basiselement 102, an dem die Schnittstelle 100 vorgesehen ist und über das das Bediengerät 42 an dem Haltearm 50 befestigt wird. An dem Basiselement 102 ist eine Schwenkeinheit 104 um eine erste Drehachse 106 drehbar gelagert. Die Lagerung erfolgt hierbei insbesondere über zwei Dünnringlager 108, 110.

An dem dem Basiselement 102 abgewandten Ende der Schwenkeinheit 104 ist ein Gehäuse 112 angeordnet, welches um eine zweite Drehachse 114 drehbar an der Schwenkeinheit 104 gelagert ist. Die Drehbarkeit wird wiederum durch die Lagerung des Gehäuses über zwei Dünnringlager 116, 118 erreicht.

Das Gehäuse 112 umfasst zwei Ringe 120, 122, zwischen denen ein scheibenförmiges Bedienelement 124 angeordnet ist, wobei dieses Bedienelement 124 relativ zu dem Gehäuse 112 und somit zu den beiden Ringen 120, 122 um eine dritte Drehachse 126 drehbar gelagert ist.

An den beiden Seitenflächen 128, 130 des scheibenförmigen Grundkörpers des Bedienelements 124 sind zwei Laschen 132, 134 angeordnet, durch die die Bedienperson zwei Finger hindurchführen kann. Insbesondere dienen die Laschen 132, 134 zur Aufnahme des Daumens und des Mittelfingers. Die Laschen 132, 134 sind über schwenkbare Pinzettenarme 133, 135 mit jeweils einer Seitenfläche 128, 130 des scheibenförmigen Grundkörpers des Bedienelements 124 verbunden. An der Umfangsfläche 136 des Bedienelements 124 ist ein Schiebetaster 138 vorgesehen, welcher bei bestimmungsgemäßem Griff des Bedienelements 124 über den aufliegenden Zeigefinger betätigbar ist. Der Schiebetaster 138 kann mit dem Bedienelement 124 derart verbunden sein, dass eine Drehung des Bedienelements 124 um Drehachse 126 eine Drehung des Schiebetasters 138 um denselben Winkel entlang der Umlauffläche 136 hervorruft, sodass die Pinzettenarme 133, 135 und der Schiebetasters 138 in einer festen räumlichen Beziehung stehen, wie dies auch aus einem Vergleich der Figur 2 mit der Figur 3 oder mit der Figur 5 hervorgeht.

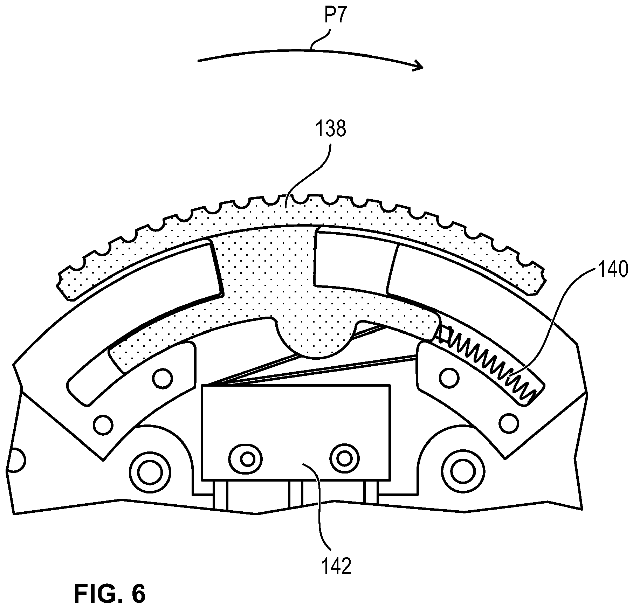

Wie in Figur 6 gezeigt, ist der Schiebetaster 138 über eine Druckfeder 140 in einer ersten Position vorgespannt und kann entgegen der Federkraft der Druckfeder 140 in Richtung des Pfeils P7 bewegt werden. Hierdurch wird ein Miniaturschalter 142 betätigt, wodurch eine zugeordnete Funktion des Manipulators ausgelöst wird. Nach Loslassen des Schiebetasters 138 wird dieser durch die Druckfeder 140 wieder in die erste Position zurück bewegt.

Das Bedienelement 140 ist somit um die drei Drehachsen 106, 114 und 126 drehbar, wobei die drei Drehachsen 106, 114, 126 jeweils orthogonal zueinander ausgerichtet sind und sich in einem gemeinsamen Schnittpunkt 150 schneiden. Der Drehbereich der Drehung des Bedienelements 140 um jeder der drei Drehachsen 106, 114, 116 beträgt vorzugsweise 360 Grad. Der Drehbereich kann bei anderen Ausführungsformen auch begrenzt sein, beispielsweise auf 90 Grad. Diese Begrenzung kann beispielsweise mit Hilfe eines Anschlags 107 erfolgen.

Mithilfe von Sensoren wird die jeweilige Drehstellung des Bedienelements 124 bezogen auf die drei Achsen 106, 114, 126 ermittelt und der Manipulator wird derart angesteuert, dass die chirurgischen Instrumente entsprechend bewegt werden.

Durch das Anordnen der drei Drehachsen 106, 114, 126 derart, dass sie sich in einem gemeinsamen Schnittpunkt 150 schneiden, wird eine besonders intuitiven Steuerung ermöglicht, die dem Chirurgen das Gefühl gibt, als ob er das chirurgische Instrument direkt in der Hand halten würde. Dieses Gefühl wird weiter dadurch unterstützt, dass dieser Schnittpunkt 150 innerhalb der Bedienelement 124 zwischen den beiden Laschen 132, 134 liegt und insbesondere mit dem Mittelpunkt des Bedienelements 124 zusammenfällt. Ferner wird hierdurch auch das auszugleichende Massenträgheitsmoment minimiert.

An dem Basiselement 102 ist ein erster Aktor vorgesehen, der über ein erstes Zahnriemengetriebe 166 mit der Schwenkeinheit 104 gekoppelt ist, so dass die Schwenkeinheit 104 über diesen ersten Aktor 160 um die erste Drehachse 106 gedreht werden kann.

An der Schwenkeinheit 104 wiederum ist ein zweiter Aktor 162 vorgesehen, welcher über ein zweites Zahnriemengetriebe 168 mit dem Gehäuse 112 gekoppelt ist, so dass das Gehäuse 112 relativ zur Schwenkeinheit 104 um die zweite Drehachse 114 gedreht werden kann.

Innerhalb des Bedienelements 124 ist ein dritter Aktor 164 vorgesehen, welcher drehfest mit dem Bedienelement 124 verbunden ist und mit diesem jeweils mitbewegt wird. Der dritte Aktor 164 ist über ein Ritzel 170 mit einem Kronenrad 172 im Eingriff, welches wiederum fest mit dem Gehäuse 112 verbunden ist. Hierüber kann das Bedienelement 124 um die dritte Drehachse 126 relativ zum Gehäuse 112 gedreht werden.

Über diese drei Aktoren 160 bis 164 kann somit zum einen das Bedienelement 124 jeweils wieder in eine voreingestellte Nullposition ausgerichtet werden. Zum anderen kann hierdurch ein Force Feedback erfolgen, bei dem die auf die chirurgischen Instrumente des Manipulators einwirkenden Kräfte und/oder Drehmomente ermittelt und über die Aktoren 160 bis 164 auf das Bedienelement übertragen werden, so dass der Chirurg ein Feedback von den ausgeführten Manipulationen erhält und somit eine realistische Operationsbedingung abgebildet wird, wie wenn der Chirurg die chirurgischen Instrumente direkt halten würde.

Die beiden Flächen 128, 130, an denen die Laschen 132, 134 befestigt sind, sind relativ zu den anderen Elementen der Bedieneinheit 124 und dem Gehäuse 112 seitlich abklappbar, wie dies in Figur 4 gezeigt ist. Hierüber wird ein Pinzettengriff gebildet, dessen Stellung entsprechend über Sensoren ermittelt wird und auch auf die Ansteuerung der chirurgischen Instrumente über den Manipulator übertragen wird.

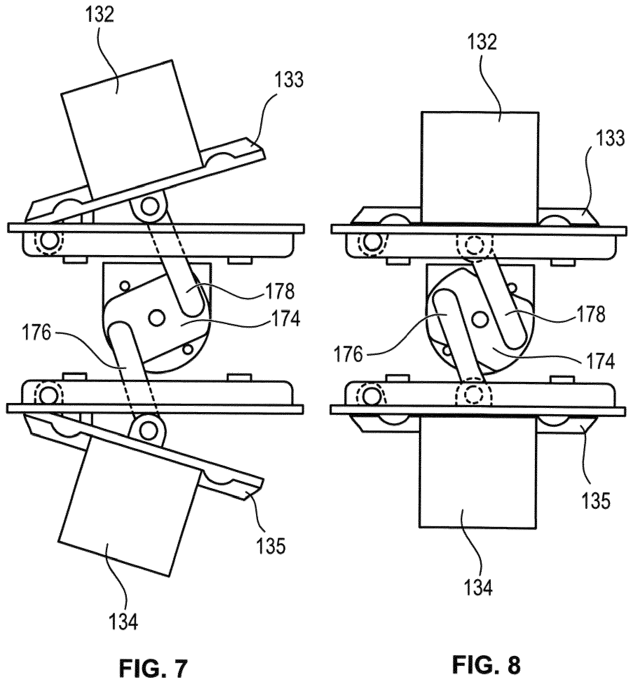

In Figur 7 und Figur 8 sind jeweils die beiden Flächen 128 und 130 und die beiden Laschen 132, 134 gezeigt, wobei in Figur 8 die Nullposition gezeigt ist, bei der die beiden Flächen 128 und 130 parallel zueinander ausgerichtet sind, und in Figur 7 die maximale Öffnung des Patientengriffes gezeigt ist, bei der die beiden Flächen 128, 130 jeweils insbesondere um einen maximalen Winkel von 17 Grad relativ zur Nullposition verschwenkt sind.

Die beiden Laschen 132, 134 sind jeweils über eine Stange 176, 178 an einer drehbaren Scheibe 174 gekoppelt, wodurch erreicht wird, dass die beiden Laschen 132, 134 immer um den gleichen Winkel relativ zur Nullposition geöffnet sind. Beim Öffnen und Schließen des Pinzettengriffs wird ein mit der Scheibe 174 gekoppelter Magnet eines Hall-Sensors gedreht, durch den die jeweilige Winkelstellung des Pinzettengriffs bestimmt und entsprechend für die Ansteuerung des Manipulators zugrunde gelegt werden kann.

### Bezugszeichenliste

- 10: System
- 12: Manipulator
- 14: Stativ
- 16, 16a bis 16d: Manipulatorarm
- 18: Patient
- 20: Stativkopf
- 24: Stativfuß
- 28: Stativarm
- 30: Zieloperationsgebiet
- 31: Mittelpunkt des Zieloperationsgebiet
- 32: Operationstischsäule
- 34: Operationstisch
- 36: Steuereinheit des Operationstisches
- 38: Patientenlagerfläche
- 40: zentrale Steuereinheit der Vorrichtung
- 41: Ausgabeeinheit
- 42: Bediengerät
- 46: Steuereinheit des Manipulators
- 47: Ausgabeeinheit
- 50: Haltearm
- 52: Drehpunkt
- 100: Schnittstelle
- 102: Basiselement
- 104: Schwenkeinheit
- 106, 114,126: Drehachse
- 107: Anschlag
- 108, 110, 116, 118: Dünnringlager
- 112: Gehäuse
- 120, 122: Ring
- 124: Bedienelement
- 128,130: Außenfläche
- 132, 134: Lasche
- 133, 135: Pinzettenarm
- 136: Umfangsfläche
- 138: Schiebeschalter
- 140: Druckfeder
- 142: Miniaturschalter
- 150: Schnittpunkt
- 160, 162,164: Aktor
- 166, 168: Zahnriemengetriebe
- 170: Ritzel
- 172: Kronenrad
- 174: Scheibe
- 176, 178: Stange
- P1 bis P7: Richtung

## Patentansprüche

1. Bediengerät für ein robotergestütztes Chirurgiesystem,
mit einem manuell betätigbaren Bedienelement (124), das zwei Laschen (132, 134) zur Aufnahme jeweils eines Fingers einer Bedienperson umfasst, und
mit einer Schnittstelle (100) zur Befestigung des Bediengeräts (42) an einer Halteeinheit (50),
wobei das Bedienelement (124), wenn das Bediengerät (42) an der Halteeinheit (50) befestigt ist, relativ zur Schnittstelle (100) um eine erste Drehachse (106), eine zweite Drehachse (114) und eine dritte Drehachse (126) drehbar ist, wobei die drei Drehachsen (106, 114, 126) jeweils orthogonal zueinander stehen,
wobei die drei Drehachsen (106, 114, 126) sich in einem gemeinsamen Schnittpunkt (150) schneiden,
**dadurch gekennzeichnet, dass** das Bedienelement (124) einen scheibenförmigen Grundkörper umfasst, und dass die beiden Laschen (132, 134) an den beiden Seiten (128, 130) des Grundkörpers angeordnet sind.

2. Bediengerät (42) nach Anspruch 1, **dadurch gekennzeichnet, dass** der Schnittpunkt (150) zwischen den beiden Laschen (132, 134), insbesondere mittig zwischen den beiden Laschen (132, 134), liegt.

3. Bediengerät (42) nach einem der vorhergehenden Ansprüche, **dadurch gekennzeichnet, dass** der Schnittpunkt (150) mit dem Mittelpunkt des Bedienelements (124) zusammenfällt.

4. Bediengerät (42) nach einem der vorhergehenden Ansprüche, **dadurch gekennzeichnet, dass** das Bediengerät (42) ein Basiselement (102), das die Schnittstelle (100) umfasst, eine relativ zum Basiselement (102) um die erste Drehachse (106) drehbar an dem Basiselement (102) befestigte Schwenkeinheit (104), und ein relativ zur Schwenkeinheit (104) um die zweite Drehachse (114) drehbar an der Schwenkeinheit (104) befestigtes Gehäuse (112) umfasst, und dass das Bedienelement (124) um die dritte Drehachse (126) drehbar an dem Gehäuse (112) gelagert ist.

5. Bediengerät (42) nach einem der vorherigen Ansprüche, **dadurch gekennzeichnet, dass** das Gehäuse (112) zwei Ringe (120, 122) umfasst, zwischen denen das Bedienelement (124) angeordnet ist, und dass das Bedienelement über ein Dünnringlager an der Gehäuse (112) drehbar gelagert ist.

6. Bediengerät (42) nach einem der Ansprüche 4 oder 5, **dadurch gekennzeichnet, dass** ein erster Aktor (160), insbesondere eine erster Motor, zum Drehen der Schwenkeinheit (104) um die erste Drehachse (106), ein zweiter Aktor (162), insbesondere ein zweiter Motor, zum Drehen des Gehäuses (112) um die zweite Drehachse (114) und/oder ein dritter Aktor (164), insbesondere ein dritter Motor, zum Drehen des Bedienelements (124) um die dritten Drehachse (126) vorgesehen sind.

7. Bediengerät (42) nach Anspruch 6, **dadurch gekennzeichnet, dass** der erste Aktor (160) am Basiselement (102), dass der zweite Aktor (162) an der Schwenkeinheit (104), und/oder dass der dritte Aktor (164) am Bedienelement (124) angeordnet ist.

8. Bediengerät (42) nach einem der Ansprüche 6 oder 7, **dadurch gekennzeichnet, dass** der dritte Aktor (164) relativ zum Bedienelement (124) drehfest im Inneren des Bedienelements (124) angeordnet ist.

9. Bediengerät (42) nach einem der vorhergehenden Ansprüche, **dadurch gekennzeichnet, dass** ein erster Sensor zur Ermittlung der Drehstellung um die erste Drehachse (106), ein zweiter Sensor zur Ermittlung der Drehstellung um die zweite Achse (114) und/oder ein dritter Sensor zur Ermittlung der Drehstellung um die dritte Drehachse (126) vorgesehen sind.

10. Bediengerät (42) nach einem der vorhergehenden Ansprüche, **dadurch gekennzeichnet, dass** das Bedienelement (124) um die zweite Drehachse (114) und/oder die dritte Drehachse (126) jeweils um 360° drehbar ist.

11. Bediengerät (42) nach einem der vorhergehenden Ansprüche, **dadurch gekennzeichnet, dass** das Bedienelement (124) einen Taster (138), insbesondere einen Schiebetaster, umfasst.

12. Bedienelement (42) nach Anspruch 11, **dadurch gekennzeichnet, dass** der Taster (138) zusammen mit den an beiden Seiten des Grundkörpers des Bedienelements (124) angeordneten Laschen (132, 134) drehbar ist.

13. Bediengerät (42) nach einem der vorhergehenden Ansprüche, **dadurch gekennzeichnet, dass** die beiden Laschen (132, 134) um einen vorbestimmten Winkel voneinander weg schwenkbar sind, so dass ein Pinzettengriff gebildet ist.

14. Bedienelement (42) nach Anspruch 13, **dadurch gekennzeichnet, dass** die beiden Laschen (132, 134) derart mit einander gekoppelt sind, dass sie relativ zu einer Nullposition immer um den gleichen Winkel geöffnet sind.

## Claims

1. An operating device for a robot-assisted surgery system,
with a manually actuatable operating element (124) comprising two straps (132, 134) for receiving one finger of a user each, and
with an interface (100) for attaching the operating device (42) to a holding unit (50),
wherein the operating element (124), when the operating device (42) is attached to the holding unit (50), is rotatable relative to the interface (100) about a first axis of rotation (106), a second axis of rotation (114) and a third axis of rotation (126), the three axes of rotation (106, 114, 126) each being orthogonal to each other,
wherein the three axes of rotation (106, 114, 126) intersect in a common point of intersection (150),
**characterized in that** the operating element (124) comprises a disk-like basic body, and that the two straps (132, 134) are arranged on both sides (128, 130) of the basic body.

2. The operating device (42) according to claim 1, **characterized in that** the point of intersection (150) lies between the two straps (132, 134), in particular centrally between the two straps (132, 134).

3. The operating device (42) according to one of the preceding claims, **characterized in that** the point of intersection (150) coincides with the center of the operating element (124).

4. The operating device (42) according to one of the preceding claims, **characterized in that** the operating device (42) comprises a base element (102) comprising the interface (100), a pivot unit (104) mounted to the base element (102) rotatably relative to the base element (102) about the first axis of rotation (106), and a housing (112) mounted to the pivot unit (104) rotatably relative to the pivot unit (104) about the second axis of rotation (114), and that the operating element (124) is mounted to the housing (112) rotatably about the third axis of rotation (126).

5. The operating device (42) according to one of the preceding claims, **characterized in that** the housing (112) comprises two rings (120, 122) between which the operating element (124) is arranged and that the operating element is rotatably mounted to the housing (112) via a thin ring bearing.

6. The operating device (42) according to one of the claims 4 or 5, **characterized in that** a first actor (160), in particular a first motor, is provided for rotating the pivot unit (104) about the first axis of rotation (106), a second actor (162), in particular a second motor, is provided for rotating the housing (112) about the second axis of rotation (114), and/or a third actor (164), in particular a third motor, is provided for rotating the operating element (124) about the third axis of rotation (126).

7. The operating device (42) according to claim 6, **characterized in that** the first actor (160) is arranged on the base element (102), that the second actor (162) is arranged on the pivot unit (104) and/or that the third actor (164) is arranged on the operating element (124).

8. The operating device (42) according to one of the claims 6 or 7, **characterized in that** the third actor (164) is arranged inside the operating element (124) in a rotationally fixed manner relative to the operating element (124).

9. The operating device (42) according to one of the preceding claims, **characterized in that** a first sensor for determining the rotational position about the first axis of rotation (106), a second sensor for determining the rotational position about the second axis (114) and/or a third sensor for determining the rotational position about the third axis of rotation (126) are provided.

10. The operating device (42) according to one of the preceding claims, **characterized in that** the operating element (124) is rotatable about the second axis of rotation (114) and/or the third axis of rotation (126) by 360° each.

11. The operating device (42) according to one of the preceding claims, **characterized in that** the operating element (124) comprises a button (138), in particular a sliding button.

12. The operating device (42) according to claim 11, **characterized in that** the button (138) is rotatable together with the straps (132, 134) arranged on both sides of the basic body of the operating element (124).

13. The operating device (42) according to one of the preceding claims, **characterized in that** the two straps (132, 134) are pivotable away from each other by a predetermined angle so that a pincer grasp is formed.

14. The operating device (42) according to claim 13, **characterized in that** the two straps (132, 134) are coupled to each other such that they are always opened by the same angle relative to a zero position.

## Revendications

1. Appareil de commande pour un système de chirurgie robotique,
avec un élément de commande (124) pouvant être actionné manuellement, qui comprend deux languettes (132, 134) destinées à loger respectivement un doigt d'un opérateur, et
avec une interface (100) pour la fixation de l'appareil de commande (42) sur une unité de retenue (50),
dans lequel l'élément de commande (124), lorsque l'appareil de commande (42) est fixé sur l'unité de retenue (50), peut être amené en rotation par rapport à l'interface (100) autour d'un premier axe de rotation (106), d'un deuxième axe de rotation (114) et d'un troisième axe de rotation (126), dans lequel les trois axes de rotation (106, 114, 126) se tiennent respectivement perpendiculairement les uns aux autres,
dans lequel les trois axes de rotation (106, 114, 126) se coupent en un point d'intersection (150) commun,
**caractérisé en ce que** l'élément de commande (124) comprend un corps de base en forme de disque, et que les deux languettes (132, 134) sont disposées sur les deux faces (128, 130) du corps de base.

2. Appareil de commande (42) selon la revendication 1, **caractérisé en ce que** le point d'intersection (150) se situe entre les deux languettes (132, 134), en particulier de manière centrale entre les deux languettes (132, 134).

3. Appareil de commande (42) selon l'une quelconque des revendications précédentes, **caractérisé en ce que** le point d'intersection (150) coïncide avec le centre de l'élément de commande (124).

4. Appareil de commande (42) selon l'une quelconque des revendications précédentes, **caractérisé en ce que** l'appareil de commande (42) comprend un élément de base (102), qui comprend l'interface (100), une unité de pivotement (104) fixée sur l'élément de base (102) de manière à pouvoir tourner autour du premier axe de rotation (106) par rapport à l'élément de base (102), et un boîtier (112) fixé sur l'unité de pivotement (104) de manière à pouvoir tourner autour du deuxième axe de rotation (114) par rapport à l'unité de pivotement (104), et que l'élément de commande (124) est monté sur le boîtier (112) de manière à pouvoir tourner autour du troisième axe de rotation (126).

5. Appareil de commande (42) selon l'une quelconque des revendications précédentes, **caractérisé en ce que** le boîtier (112) comprend deux bagues (120, 122), entre lesquelles l'élément de commande (124) est disposé, et que l'élément de commande est monté à rotation sur le boîtier (112) par l'intermédiaire d'un palier à bagues minces.

6. Appareil de commande (42) selon l'une quelconque des revendications 4 ou 5, **caractérisé en ce qu'**un premier actionneur (160), en particulier un premier moteur, destiné à faire tourner l'unité de pivotement (104) autour du premier axe de rotation (106), un deuxième actionneur (162), en particulier un deuxième moteur, destiné à faire tourner le boîtier (112) autour du deuxième axe de rotation (114) et/ou un troisième actionneur (164), en particulier un troisième moteur, destiné à faire tourner l'élément de commande (124) autour du troisième axe de rotation (126), sont prévus.

7. Appareil de commande (42) selon la revendication 6, **caractérisé en ce que** le premier actionneur (160) est disposé sur l'élément de base (102), que le deuxième actionneur (162) est disposé sur l'unité de pivotement (104), et/ou que le troisième actionneur (164) est disposé sur l'élément de commande (124) .

8. Appareil de commande (42) selon l'une quelconque des revendications 6 ou 7, **caractérisé en ce que** le troisième actionneur (164) est disposé à l'intérieur de l'élément de commande (124) de manière solidaire en rotation par rapport à l'élément de commande (124).

9. Appareil de commande (42) selon l'une quelconque des revendications précédentes, **caractérisé en ce qu'**un premier capteur destiné à déterminer la position de rotation autour du premier axe de rotation (106), un deuxième capteur destiné à déterminer la position de rotation autour du deuxième axe (114) et/ou un troisième capteur destiné à déterminer la position de rotation autour du troisième axe de rotation (126) sont prévus.

10. Appareil de commande (42) selon l'une quelconque des revendications précédentes, **caractérisé en ce que** l'élément de commande (124) peut être amené en rotation autour du deuxième axe de rotation (114) et/ou du troisième axe de rotation (126) respectivement de 360°.

11. Appareil de commande (42) selon l'une quelconque des revendications précédentes, **caractérisé en ce que** l'élément de commande (124) comprend un bouton-poussoir (138), en particulier un bouton-poussoir coulissant.

12. Appareil de commande (42) selon la revendication 11, **caractérisé en ce que** le bouton-poussoir (138) peut être amené en rotation conjointement avec les languettes (132, 134) disposées sur les deux faces du corps de base de l'élément de commande (124).

13. Appareil de commande (42) selon l'une quelconque des revendications précédentes, **caractérisé en ce que** les deux languettes (132, 134) peuvent pivoter à distance l'une de l'autre selon un angle prédéfini, de sorte qu'une pince pouce-index est formée.

14. Appareil de commande (42) selon la revendication 13, **caractérisé en ce que** les deux languettes (132, 134) sont accouplées l'une à l'autre de telle sorte qu'elles sont toujours ouvertes du même angle par rapport à une position zéro.
